# EUROPEAN PATENT APPLICATION

(11) **EP 0 635 575 A1**
(43) Date of publication of application: **25.01.1995**
(21) Application number: 94110082.8
(22) Date of filing: 29.06.1994
(51) Int. Cl.: C12P 21/08, C12N 5/20, G01N 33/573, G01N 33/574

(54) **Monoclonal antibodies against epitopes found in free but not in alpha-1-antichmotrypsin complexed prostate specific antigen**

(30) Priority: 22.07.1993 US 94901
(71) Applicant: WALLAC OY, SF-20101 Turku (FI)
(72) Inventor: Matikainen, Marja-Terttu, SF-23100 Mynämäki (FI); Lilja, Hans, S-236 00 Höllviken (SE)
(74) Representative: Dost, Wolfgang, Dr.rer.nat., Dipl.-Chem.

(57) **Abstract**

The present invention provides novel hybridoma cell lines which produce monoclonal antibodies that bind epitopes found in the free form of Prostate Specific Antigen (PSA) but not in PSA complexed to α₁-antichymotrypsin. PSA is a serine protease with a molecular mass of 33 kDa found as a specific cancer marker of prostatic cancer. The antibodies recognize their epitopes on the zymogen as well as on the active enzyme but the epitope is hidden by their specific inhibitor α₁-antichymotrypsin (ACT), normally found in human serum. The hybridomas are produced by fusing an immortal cell, a cell having the ability to replicate indefinitely in myeloma cell culture (NS-1, P3x63-Ag8,45.6 Tg), and an effector immune cell following immunization of the immune host with PSA isolated from patient material.

The monoclonal antibodies produced by the hybridoma cell lines of the present invention are useful in the detection of free form of PSA which is important in screening of the patient material to distinguish carcinoma of the prostate (CAP) from less fatal benign prostatic hyperplasia (BHP).

## Description

### FIELD OF THE INVENTION

The present invention provides novel hybridoma cell lines which produce monoclonal antibodies that bind epitopes found in free form of Prostate Specific Antigen (PSA) but not in PSA compelexed to α₁-antichymotrypsin.

### BACKGROUND OF THE INVENTION

The publications and other materials used herein to illuminate the background of the invention , and in particular, cases to provide additional details respecting the practice, are incorporated by reference.

The present invention relates to carcinoma of the prostate (CAP) that is the most common malignant disease among males in Europe and in the US. The risk to develop CAP increases significantly with age, and the lifetime risk to develop clinically significant CAP is estimated to be 10%. More than 30% of men diagnosed to have CAP will eventually die from the disease. The early detection of organ confined prostatic cancer provides on opportunity to radical treatment of disease. When the tumor is spread outside the prostate the prognosis is poor and there is no use for radical treatments. At diagnosis, CAP is found to be spread outside the gland in about 50% of cases. If cancer has metastased, the prognosis after hormone treatment is about 20-30% in five years if patient has local disease the survival is close to 60% (Blom J. Available screening tests for prostatic cancer. In: Miller A., Chamberlain J., Day N., Hakama M., propok R. ed. Cancer screening. Cambridge UICC, Cambridge University Press, 1991: 294-299). That is why it is possible to reduce death by performing a prostate cancer-related early diagnosis of the disease.

However, it has recently been shown that early detection of CAP can be made using procedure that includes serum PSA measurement, digital rectal examination (DRE) and transrectal ultrasonography (Catalona WJ., Smith DS., Ratliff TL., Dodds KM., Coplen DE., Yuan JJ., et al. Measurement of prostate-specific antigen in serum as a screening test for prostate cencer. NEngl J Med 1991; 324: 1156-61; Chadwick DJ., Kemple T., Astley JP., MacIver AG., Gillatt DA., Adams P., Gingell JC. Pilot study of screening for prostate cancer in general practice. Lancet 1991; 338: 613-6).

Low levels of prostate derived PSA (about 10⁻⁶ of that in the seminal fluid) normally occur in serum from adult males, but abnormally high levels are regularly detected in prostate disease. PSA measurements are widely used to monitor disease progress, evaluate therapeutic response, and detect early recurrence after radical treatment of CAP. Above normal serum levels of PSA are also detected in a high proportion of patients with benign prostatic hyperplasia (BPH) and the above normal levels alone are specific for cancer only at high serum PSA levels. This limits ability of serum PSA measurements to detect CAP at organ contined stage of the disease.

**In vivo** it has been unequivocally shown that the predominant fraction (up to 95%) of the PSA immunoreactivity in serum is an approximately 90 kDa species of PSA in complex with α₁-antichymotrypsin (ACT) (Lilja H., Christensson A., Dahlen U., Matikainen M.-T., Nilsson O., Pettersson K., Lövgren T. Prostate-Specific Antigen in serum occurs predominantly in complex with α₁-antichymotrypsin. Clin.Chem. 1991; 37: 1618-1625; Lilja H., Cockett ATK., Abrahamsson PA. (1992) Cancer 70: 230 suppl.; Christensson A., Björk T., Nilsson O., Dahlen U., Matikainen M.-T., Cockett A.T.K., Abrahamsson P.A., Lilja H. Serum prostate-specific antigen complexed to α₁-antichymotrypsin as an indicatorof prostate cancer. (1993) J.Urol. 150, in press.; Stenman U.-H., Leinonen J., Alftan H., Rannikko S., Tuhkanen K., Alftan O. 1991 Cancer Res. 51: 222-226). This conclusion has been supported by data reported by Wood et al. (Wood W.G., van der Sloot E., Böhle A. 1991, Eur.J.Clin.Chem.Clin.Biochem. 29: 787).

It is also shown that a 25- to 40-kDa PSA species not associated to ACT constitutes a minor fraction of the PSA immunoreactivity in serum although serum ACT occurs at 1,000 fold molar excess to this form of PSA (Lilja et al 1991). Therefore it is unlikely that the free form of serum PSA manifests enzyme activity.

A greater proportion of serum PSA is complexed to ACT in patients with CAP compared to that with BPH. In agreement with this, the 25- to 40-kDa non-complexed PSA was a smaller proportion of PSA in serum patients with CAP than in the BPH patients (Christensson A., Björk T., Nilsson O., Dahlen U., Matikainen M.-T., Cockett A.T.K., Abrahamsson P.A., Lilja H. 1993, J.Urol. 150; in press., Stenman U.H., Leinonen J., Alftan H., Rannikko S., Tuhkanen K., Alftan O. A complex between prostate-specific antigen and α₁-antichymotrypsin in the major form of prostate-specific antigen in serum of patients with prostatic cancer:assay of the complex improves clinical sensitivity for cancer. Cancer Res. 1991; 51: 222-226). These differences are a) valid at a statistical level of very high significance (p<0.001), b) valid at PSA concentrations in serum below 10 ng/ml, and c) unrelated to the total concentration of PSA in serum. The diagnostic specificity of the procedures at PSA-levels below 10 ng/ml a ratio <0.18 in non-complexed PSA to total PSA was used to indicate malignant potential of prostate disease. A significant increase in diagnostic specificity at sensitivity levels of 90% reached almost 75% by new procedures compared to a specificity of 55% for the conventional assays (Christensson et al. 1993). The measurement of PSA-ACT and PSA at the same time, gives 2 to 3 times higher cancer specificity when compared to the measurement of the complex alone. This screening method decreases the amount of false positive cases to half of that found in BPH (Stenman UH., Leinonen J., Alftan H., Rannikko S., Tuhkanen K., Alftan O. A complex between prostate-specific antigen and α₁-antichymotrypsin in the major form of prostate-specific antigen in serum of patients with prostatic cancer: assay of the complex improves clinical sensitivity for cancer. Cancer Res. 1991; 51: 222-6).

PSA is a tissue-specific glycoprotein (33 kDa) originally described and characterized by several independent groups (Sansabaugh GF. 1978 Isolation and characterization of a semen-specific protein from human seminal plasma: a potential new marker for semen identification. J. Forsensic. Sci. 23: 106-115; Wang MC., Valenzuela LA., Murphy GP., Chu TM. 1979. Purification of a human prostate gland. Invest.Urol. 17: 159-163, Hara M., Inoue T., Koyoanagi Y., Fukuyama T., Iki H. 1969. Nippon Holgaku Zasshi 23:333, Papsidero LD., Kuriyama M., Wang MC., Horoszewicz J., Leong SS., Valenzuela L., Murphy GP., Chu TM. 1981 Prostate antigen: a marker for human epithelial cells. J.Natl. Cancer Inst. 66: 37).

PSA manifests serine protease activity and a number of characteristics similar to that of members in the family of glandular kallikreins. In the prostate epithelium, PSA is produced as a precursor (Lundwall A., Lilja H. 1987. Molecular cloning of human prostate-specific antige cDNA FEBS Lett 214: 317-322, Lundwall A. 1989. Biochem Biophys. Res. Commun. 161: 1151). The zymogen is converted into active PSA after trypsin-like peptide bond cleavage that results in the release of a small amino-terminal peptide from the zymogen.

The primary structure of mature PSA contains 237 amino acid residues and one single site for asparagine-linked carbohydrate attachment (Watt KWK., Lee P-JL., M'Timkulu TM., Chan W-P., Lool R. 1986. Human prostate-specific antigen: structural and functional similarity with serine proteases. Proc Natl Acad Sci (USA) 83:3166-70, Lundwall A., Lilja H. 1987. Molecular cloning of human prostate-specific antigen cDNA FEBS Lett 214:317-322, Schaller J., Akiyama K., Tsuda R., Hara M., Marti T., Rickli E. 1987. Isolation, characterization and amino-acid sequence of gamma-seminoprotein, a glycoprotein from human seminal plasma. Eur.J.Biochem. 170:111-120). PSA displays restricted chymotrypsin-like substrate specificity, i.e. cleaves peptide bonds carboxy-terminal of bulky hydrophobic residues like tyrosine and leucine. In freshly ejaculated human semen, semenogelin I and II, and fibronectin become digested by PSA. These are seminal vesicle-secreted protein substrates to PSA that constitute major structural proteins in the loose sperm-entrapping gel formed at the ejaculatory mixing of secretions from the accessory sex glands. Proteolysis results in dissolution of the gel and release of progressively motile spermatozoa(Lilja H. 1985 A kallikrein-like serine protease in prostatic fluid cleaves the predominant seminal vesicle protein. J.Clin.Invest. 76:1899-1903, Lilja H., Oldbring J., Rannevik G., Laurell C.-B. 1987 Seminal vesicle-secreted proteins and their reactions during gelation and liquefaction of human semen. J.Clin.Invest. 80:281, McGee RS., Herr JC 1988. Human seminal vesicle-specific antigen is a substrate for prostate-specific antigen (orP-30). Biol.Repro 39: 499-510).

### SUMMARY OF THE INVENTION

The present invention provides novel hybridoma cell lines which produce monoclonal antibodies (Mabs) that bind epitopes found in the free form of Prostate Specific Antigen (PSA) not accessible in PSA-ACT complex. The present invention further relates to the Mabs produced by theses cell lines. The Mabs of the present invention are useful in the diagnostic assay described in WO 92/01936 and in U.S. Application Serial No. 07/956,509, filed 21 January 1993, incorporated herein by reference.

The hybridomas are produced by fusing an immortal cell, a mouse myeloma cell, having the ability to replicate indefinitely in cell culture, and an effector immune cell following immunization of the immune cell host with an isolated PSA (Christensson A et al., 1990). Enzymatic activity of prostate-specific antigen and its reactions with extracellular serine proteinase inhibitors. Eur. J. Biochem. 194: 755 - 763). A cell hybrid made by fusing normal spleen cells to plasmacytoma or myeloma cell line can provide a continuous source of antibody of predefined specificity - based on the immunization.

The monoclonal antibodies produced by the hybridoma cell lines of the present invention are useful for the detection and measurement of the free, 25 kDa to 40 kDa subfraction of PSA not comlexed to ACT. Thus, the Mabs of the present invention are capable of distinguishing free PSA from PSA complexed to ACT in serum.

### BRIEF DESCRIPTION OF THE DRAWINGS

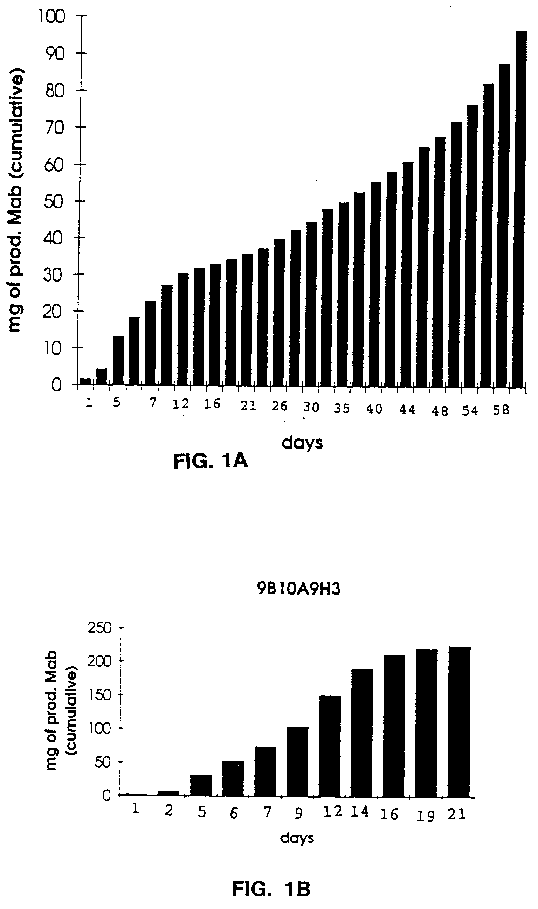

**Figure 1A** shows the production of monoclonal antibody (Mab) 5A10E7F11H4 **in vitro** in the hollow fiber system Technomouse made by Technomara. In the intracapillary space (IC) the Dulbecco's essential medium (with high glucose 4.5 g/L and Na-bicarbonate 2.2 g/L) was used. In the extracapillary space (EC) with the cells was the same medium but supplemented with 2.5% foetal calf serum. Mab from the EC space was harvested three times a week replacing the space with same volume (10 ml) of fresh medium. The results are shown as cumulative, as mg produced Mab in ratio to time cultured. The numbers on the X-axis below the columns indicate the days passed after the inoculation.

**Figure 1B** shows the production of monoclonal antibody (Mab) 9B10A9H3 **in vitro** in the hollow fiber system Technomouse made by Technomara. In the intracapillary space (IC) the Dulbecco's essential medium (with high glucose 4.5 g/L and Na-bicarbonate 2.2 g/L) was used. In the extracapillary space (EC) with the cells was the same medium but supplemented with 2.5% foetal calf serum. Mab from the EC space was harvested three times a week replacing the space with same volume (10 ml) of fresh medium. The results are shown as cumulative, as mg produced Mab in ratio to time cultured. The numbers on the X-axis below the columns indicate the days passed after the inoculation.

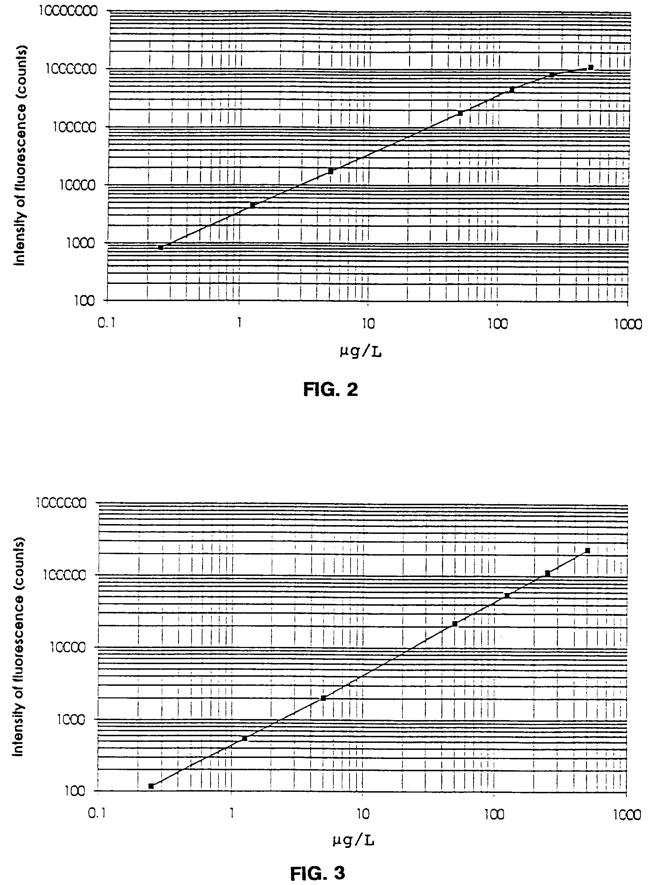

**Figure 2** depicts the standard curve of Sandwich-type immuno-fluorometric assay (IFMA) for antiPSA 5A10E7F11H4. As standard the affinity purified antiPSA 5A10E7F11H4 produced earlier was used. The linear range reached from about 0.25 ng/ml to 250 ng/ml. The solid-phase precoated with rabbit antimouse immunoglobulin (Ig) captured the monoclonal antibodies (Mabs) to be tested in different dilutions on overnight incubation at +4°C. The concentration of specific Mabs in samples was identified through incubation with a fixed amount (excess) amount of Eu-labelled PSA followed with the measurement of fluorescence. The intensity of fluorescence is in ratio to the amount of bound Mab.

**Figure 3** shows the standard curve of IFMA for antiPSA 9B10A9H3. As the standard affinity purified antiPSA 9B10A9H3 produced earlier was used. The linear range reached from about 0.25 ng/ml to 500 ng/ml. The cell culture supernatants with unknown concentrations of antiPSA 9B10A9H3 Mabs were incubated in microtiter plate wells precoated with rabbit antimouse Ig (overnight, +4°C). The amount of Mab was identified through incubation with a fixed amount (excess) amount of Eu-labelled PSA following with the measurement of intensity of fluorescence.

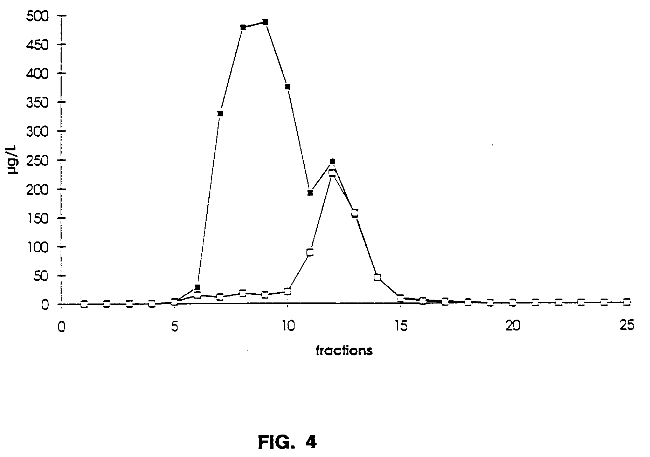

**Figure 4** shows the fractionation of PSA mixture that contains PSA-ACT complex and free PSA. It was made in Ultrogel 44 column using 50 mmol/L Tris buffer pH 7.2 with 0.15 mol/L NaCl. The total PSA (closed squares) (PSA + PSA-ACT) was measured with the specific PSA kit (DELFIA™, Wallac). Free form of PSA was measured using antiPSA 5A10E7F11H4 as catching antibody on the solid phase (Lövgren et al. (1984). Determination of hormones by Time-Resolved Fluoroimmunoassay. Talanta 31: 909 - 916) and Eu-labelled antiPSA Mab that recognizes both the free form and complexed form of PSA as detector antibody (the DELFIA™-kit reagent) (open squares).

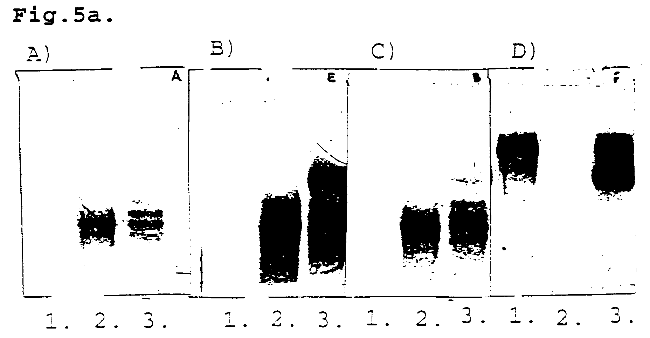

**Figure 5a** shows the ability of antiPSA Mabs 5A10E7F11H4 and 9B10A9H3 to recognize PSA as native form. It was determined after blotting the proteins PSA, PSA-ACT and ACT onto polyvinylidene fluoride, (PVDF) membrane following agarose electrophoresis using 12% homogeneous gels in 75 mmol/L barbital buffer containing Ca²⁺ 2 mmol/L at pH 8.6.

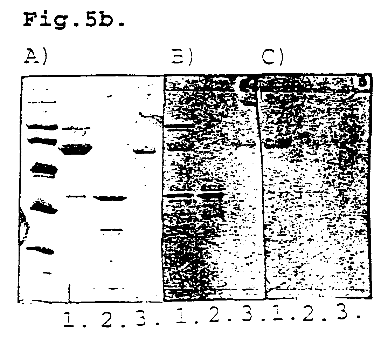

Immunoreactivity of Mabs: A) 5A10E7F11H4 and C) 9B10A9H3 was detected through incubation with secondary antibody alkaline phosphatase (AP) conjugated goat antimouse IgG and substrate (NCB,BCIP). Used as positive control was antiPSA Mabs 2E9 (B) that detects both free and complex forms of PSA as well as antiPSA 9F10 (D) that detects ACT part of PSA/ACT complex and free form of ACT.
Lane 1. 4 µg of purified ACT
Lane 2. 2 µg of purified PSA
Lane 3. 2/6 µg PSA/ACT complex
**Figure 5b** shows the ability of antiPSA Mabs against the free of PSA to recognize the specific epitopes after reduction with 10 mmol/L ditiothreitol (DTT) and carboxymethylation of PSA. It was made using semi-dry electroblotting onto PVDF membrane after SDS-PAGE (the sodium dodecyl sulphate polyacrylamide gel electrophoresis) in 12% gels.

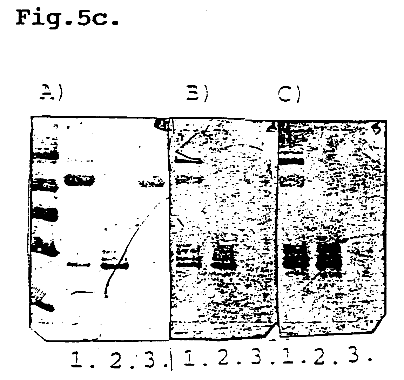

Immunoreactivity of Mabs: A) the left lane contains low molecular weight control and the PSA antigens as Coomassie Brilliant Blue stained, B) antiPSA Mabs 2E9 as positive control that detects both free and complex forms of PSA and C) 9B10A9H3 was detected through incubation with AP-conjugated goat antimouse IgG and substrate.
Lane 1. 1/3 µg PSA/ACT complex
Lane 2. 1 µg of purified PSA
Lane 3. 2 µg of purified ACT
**Figure 5c** shows the ability of antiPSA 5A10E7F11H4 and 9B10A9H3 to identify the specific epitopes after SDS-PAGE (12% in non-reduced conditions) and Western blotting onto PVDF membrane.

Immunoreactivity of Mabs: A) the left lane contains low molecular weight control and the PSA antigens as Coomassie Brilliant Blue stained, B) antiPSA 5A10E7F11H4 and C) antiPSA 9B10A9H3 was identified through incubation with AP-conjugated goat antimouse IgG and substrate.
Lane 1. 1/3 µg PSA/ACT complex
Lane 2. 1 µg of purified PSA
Lane 3. 2 µg of purified ACT

### DETAILED DESCRIPTION OF THE PREFERRED EMBODIMENTS

The present invention provides novel hybridoma cell lines which produce monoclonal antibodies (Mabs) that bind epitopes found in the free form of Prostate Specific Antigen (PSA) not accessible in PSA-ACT complex. The present invention further relates to the Mabs produced by theses cell lines. The Mabs of the present invention are useful in the diagnostic assay described in WO 92/01936 and in U.S. Application Serial No. 07/956,509, filed 21 January 1993, incorporated herein by reference.

The report by Köhler and Milstein (Köhler G. and Milstein C. 1976. Eur.J.Immunol. 6:511-519. Derivation of specific antibody-producing tissue culture and tumor lines by cell fusion) that a cell hybrid made by fusing normal spleen cells with malignantly transformed antibody-secreting cells (plasmacytoma or myeloma cell line) can provide a continuous source of antibody of predefined specificity has led to the explosion of the use of Mabs.

The optimal myeloma cell line should be stable hypoxanthine-guanine-phosphoribosyltransferase (HGPRT)-negative, not producing its own immunoglobulin. Fusion is promoted using polyethyleneglycol because cells do not fuse spontaneously. Following the fusion procedure the selection of spleen-myeloma hybrids should be made through the selection with aminopterin which blocks the **de novo** synthesis of purines via inosine monophosphate and also prevents the methylation of deoxyuridine monophosphate to deoxythymidine monophosphate (Littlefield J.W. Selection of hybrids from matings of fibroblasts **in vitro** and their presumed recombinants. Science 1964; 145: 709-710). The cell in culture becomes dependent on extraneous sources of purines and thymidine which can be incorporated into new RNA and DNA via so called salvage pathways in the presence HGPRT enzyme originated from the spleen cell.

The hybridomas are produced by fusing an immortal cell, a mouse myeloma cell, having the ability to replicate indefinitely in cell culture, and an effector immune cell following immunization of the immune cell host with an isolated PSA (Christensson A et al., 1990). Enzymatic activity of prostate-specific antigen and its reactions with extracellular serine proteinase inhibitors. Eur. J. Biochem. 194: 755 - 763). A cell hybrid made by fusing normal spleen cells to plasmacytoma or myeloma cell line can provide a continuous source of antibody of predefined specificity - based on the immunization.

The monoclonal antibodies produced by the hybridoma cell lines of the present invention are useful for the detection and measurement of the free, 25 kDa to 40 kDa subfraction of PSA not comlexed to ACT. Thus, the Mabs of the present invention are capable of distinguishing free PSA from PSA complexed to ACT.

### Example 1

### Purification of proteins

**(A) PSA** isolated from plasma of patient semen was used for immunization of mice. The purification of PSA was made by Aprotinin-Sepharose chromatography in 50 mmol/L Tris pH 7.8 containing 0.5 mol/L NaCl (Christensson A., Laurell C.-B., Lilja H. 1990 Enzymatic activity of prostate-specific antigen and its reactions with extracellular serine proteinase inhibitors. Eur.J.Biochem. 194: 755-763). Purification was preceded with the procedure described by Lilja H, A kallikrein-like serine protease in prostatic fluid cleaves the predominant seminal vesicle protein, J. Clin. Invest.76: 1899 - 1903 (1985). The immunoreactivity of isolated PSA was measured by electroimmunoassay, and protease activity determined using the synthetic peptide substrates.
**(B) Antichymotrypsin** was purified according the method described by Christensson A., Laurell C.-B. and Lilja H. (Enzymatic activity of prostate-specific antigen and its reactions with extracellular serine proteinase inhibitors. Eur.J.Biochem.1990; 194: 755-763). The low-density lipoproteins were precipitated from fresh, citrated human blood plasma. The purification procedure contained the chromatographic steps in heparin-Sepharose, DEAE-Sepharose and finally in Blue-Sepharose columns following with the precipitation of ACT using ammonium sulphate precipitation. Precipitated ACT was collected, dissolved in water and stored in water containing glycine (0.2 mol/L) and Na₂EDTA (10 mmol/L) at -20°C. Purity was evaluated by agarose gel electrophoresis, sodium dodecylsulfate-polyacrylamide gel electrophoresis (SDS-PAGE), and amino-terminal sequence determination.

### Example 2

### Generation and isolation of PSA-ACT complexes

PSA-ACT complexed were generated when purified PSA and ACT were incubated in 50 mM Tris buffer pH 7.4 (4 hours, at +37°C) in molar ratio 1 to 4 (1 mg to 8 mg), respectively. The complexed form was fractionated from the free components through gel filtration in Sepharose S-200 column in the 50 mM Tris buffer but at pH 7.2. The complex containing fractions were pooled and rerun in the same column in the same conditions (Christensson A., Laurell C.-B., Lilja H. Enzymatic activity of prostate-specific antigen and its reactions with extracellular serine proteinase inhibitors. Eur.J.Biochem. 1990; 194: 755-763).

### Example 3

### Immunization procedure

The 3 months old Balb/c mice (male) were intraperitoneally immunized with purified PSA (70 µg/mouse) emulsified with Freund's complete adjuvant. Booster doses of 50 µg were given at three- to four-week intervals and a final booster after another three-week interval. Four days after the final booster dose, the mice were killed and their splenic lymphoid cells fused with plasmacytoma cells NS-1 (P3X63-Ag8,45.6Tg) at a 1:1 ratio. The fused cells were harvested in 96-well culture plates (Nunc) in Ex-Cell 320 medium (Seralab) containing fetal calf serum (FCS, 20%) and HAT supplement (hypoxanthine- aminopterin-thymidin, Gibco, 043-01060H, diluted 50-fold). Cells were cultured at +37°C, in a 5% CO₂ atmosphere. After 10 days HAT-supplemented medium was changed to HT-supplemented cell culture medium (Gibco, 043-01065H, diluted 50-fold). HT medium was identical to HAT medium but without aminopterin.

At between two to three weeks, specific antibody production was tested with antigen specific IFMA. The master clones were cloned by limited dilutions (Staszewski, R. Cloning by limiting dilution: an improved estimate that an interesting culture is monoclonal. The Yale Journal of Biology and Medicine 57 (1984), 865-868. Positive clones were expanded onto 24-well tissue culture plates (Nunc). Recloned and retested by the same method. Positive clones were tested by immunoblotting after electrophoresis in reduced and unreduced conditions. The stable clones secreted immunoglobulins belonging to the IgG class.

Two clones, designated 5A10E7F11H4 and 9B10A9H3 were found to stably secrete monoclonal antibody which was determined to be of immunoglobulin class IgG by IFMA (Clin. Chem. 1991, 37: 1618-1625, Lilja et al.) using standard methods. Hybridomas 5A10E7F11H4 and 9B10A9H3 were deposited with the European Collection of Animal Cell Cultures (ECACC), Public Health Laboratory Service, Centre for Applied Microbiology and Research, Porton Down, Salisbury, Wiltshire SP4 OJG, Great Britain on March 12, 1993, and given the ECACC accession Nos. 93031201 and 93031202, respectively.

Balb/c mice were used to produce Mabs as ascites fluid. The hybridomas were intraperitoneally (i.p.) injected into mice after the pretreatment of animals with pristane (2,6,10,14-tetramethyl-pentadecan 98%, Aldrich-Chemie D-7924 Steinheim, cat.no T 2,280-2). They were primed through injection of 0.5 ml volume of pristane (i.p.) about two weeks earlier. Amounts of cells injected were 7 to 8 x 10⁻⁶ per mouse. The resultant ascites was collected 10-14 days after injection of the hybridomas, containing on average 3 mg/ml of antibody as determined by antigen specific IFMA.

### Example 4

**In vitro** production of monoclonal antibodies was made in Hollow Fiber Bioreactors made by Technomara.
(A) Pretreatment of Technomouse-system: The bottles with caps and filters were autoclaved at 121°C and 1.1 bar pressure for one-half hour. Media bottles were filled with 1 L Dulbecco's MEM (Gibco, 042-02501, with glucose 6.4 g/L, glutamine 2 mmol/L 066-1051H, Na-pyruvate 1 mmol/L 066-1840E). The bioreactor holder was aseptically transferred in the Technomouse tray. The pump was loaded, the medium lines as well as the empty waste bottles (the outflow line) aseptically connected.
   The fill and flush program which washes all the preserving material from the Intracapillary space (IC) of the bioreactor started at flow rate 150 ml/h for 4 hours. The washing continued at flow rate 50 ml/h for 20 hours with simultaneous washing of the Extracapillary (EC) space with 5% FCS in Dulbecco's MEM (DMEM). The medium in EC space was aseptically changed to fresh medium. On the following day the bioreactor was ready for inoculation of the hybridoma cells.
(B) Harvesting of Mabs with Technomouse-system: 5 to 8 x 10⁷ cells (harvested in the normal cell culture bottles in 10% FCS - DMEM) were collected and inoculated in 5 ml volume of DMEM containing 5% FCS. The medium flow rate in IC space was 100 ml/h. The recycling method was used for harvesting Mabs: the medium line was connected to the medium bottle "out" taking the medium out from the bottle to the bioreactor IC space; the outflow line was connected to the medium bottle "in" bringing the medium back into the bottle. Mabs produced were harvested three times a week on Monday, Wednesday and Friday replacing the 10 ml volume of fresh medium containing 2.5% FCS in DMEM.
   The antibody produced in ascites fluid or in Technomousesystem was purified by Affigel Protein A MAPS II Kit (BioRad). The column was equilibrated for the purification procedure with binding buffer (pH 9.0). The antibodies from ascites fluid or in vitro supernatant were connected to the protein A -matrix in the binding buffer and washed with the binding buffer till the baseline reached (detected by recorder). The specifically bound material was eluted from protein-A with elution buffer (pH 3.0) and the fractions were collected in the tubes containing the volume of 1 mol/L Tris-HCl pH 9.0 needed to neutralize the fraction immediately. The column was regenerated with regeneration buffer and stored till next use in 50 mmol/L Na-phosphate buffer (pH 7.5) containing 0.05% NaN₃ as preservative.

### Example 5

### The IFMA-type screening assay

### (A) Labelling of PSA with Europium:

The labelling was performed according to Mukkala V.-M., Mikola H., Hemmilä I, The synthesis and use of activated N-benzyl derivatives of diethylenetriaminetetraacetic acids: alternative reagents for labeling of antibodies with metal ions. Anal.Biochem. 1989;176 (2):319-325, with slight modifications. The 25 times molar excess of isothiocyanate DTTA-Eu (N1 chelate, Wallac, Finland) was added to the PSA solution (1 mg/ml in 0.15 mol/L NaCl) and the pH was adjusted to 9.8 by adding one tenth of 0.5 mol/L sodium carbonate (Merck) buffer, pH 9.8. The labelling was performed over night at +4°C. Unbound label was removed using Ultrogel AcA 54 column (1x50 cm) (IBF, France) with TSA buffer (50 mmol/L Tris-HCl pH 7.8 containing 0.15 mol/L NaCl) as eluent. The PSA yield was calculated using the fluorescence intensity of the peak fraction in the ratio to total fluorescence measured by ARCUS-1230 (Wallac). After purification 1 mg/ml bovine serum albumin (BSA) was added to the labelled PSA and the labels were stored at +4°C.

The number of Europium ions incorporated per PSA molecule was determined by measuring the fluorescence in ratio to that of known EuCl₃ standards (Hemmilä I., Dakubu S., Mukkala V.-M., Siitari H. and Lövgren T. Europium as a label in Time-Resolved Immuno-fluorometric assay. Anal.Biochem. 1984; 137: 335-343).

### (B) The Assay Procedure:

The antibody production against PSA was screened with Sandwich type immunofluorometric assay using microtiter strip wells (Nunc, polysorb) coated with rabbit antimouse Ig (Z 259, Dakopatts, Lövgren T., Hemmilä I., Pettersson K., Eskola J.U. and Bertoft E. Determination of hormones by Time-Resolved Fluoroimmunoassay. Talanta 1984; 31 (10B): 909-916). Precoated wells were washed once with wash solution (DELFIA) by Platewash 1296-024 (Wallac). The DELFIA assay buffer was used as dilution buffer for cell culture supernatants and for standard. Mab against PSA produced by Hybritech (0812) was as standard in the preliminary screening assay at concentration between 1.6 ng/ml and 1,000 ng/ml in assay buffer (100 µl, DELFIA).

A two-hours incubation at room temperature (or alternatively an overnight incubation at +4°C) was started by shaking on Plateshake (1296-001, Wallac) for 5 minutes following by washing of four times with wash solution as before and with the addition of the labelled PSA.

The Eu-labelled PSA (4.4 Eu/PSA, Hemmilä et al., 1984) was used at concentration 30 ng/well in 100 µl of the assay buffer. After one-hour incubation at room temperature starting with 5 minutes shaking on Plateshake, the strips were washed as before.

After an hour's incubation at room temperature with the label the frame was washed four times as before with the wash solution followed with the addition of the Enhancement solution (200 µl/well, DELFIA). The plates were shaken for 5 minutes on Plateshake and the intensity of fluorescence was measured by ARCUS-1230 in 10 to 15 minutes (Lövgren T., Hemmilä I., Pettersson K. and Halonen P. Time-resolved fluorometry in immunoassay. In: Collins W.P. (ed), Alternative Immunoassays. John Wiley & Sons Ltd, 1985; 203-216).

### (C) Immunoblotting

Agarose gel electrophoresis was run in 75 mmol/L barbital buffer (pH 8.6) containing 2 mmol/L Ca⁺(Jeppsson J.-O., Laurell C.-B, Franzen B. Agarose electrophoresis. Clin. Chem. 1979, 25: 629-638). The ability of antibody to recognize PSA was determined through blotting of PSA-containing proteins from agarose gels to polyvinylidene difluoride (PVDF) membranes . SDS-PAGE was run in gradients of 70-120 or 90-170 g/L (Laemmli U.K. Cleavage of structural proteins during the assembly of the head of bacteriophage T₄. Nature (London) 1970;227:680-685) using the buffer system of Blobel (G) and Dobberstein (B) (Transfer of proteins across membranes. J.Cell.Biol. 1975; 67:835-51).

Western blotting was performed essentially as described by Burnette (WN) ("Western blotting": electrophoretic transfer of proteins from sodium dodecyl sulfate-polyacrylamide gels to unmodified nitrocellulose and radiographic detection with antibody and radioiodinated Protein A. Anal. Biochem. 1981;112:195-203) in an Ancos (Denmark) apparatus for semidry electroblotting in accordance with the manufacturers' instructions.

Immunoreacted antibodies were detected with alkaline phosphatase-conjugated goat anti-mouse IgG following the procedure recommended by the manufacturer (Promega Biotec).

### RESULTS

### (A) Production of Mabs

The antiPSA cell lines 5A10E7F11H4 and 9B10A9H3 of the present invention produced antibodies at mean concentration of 6 µg/ml and 25 µg/ml in the cell culture medium , respectively. After inoculation of the cells to their own bioreactors in Technomouse system (8x10⁷ cells/ 5A10E7F11H4; 5x10⁷ cells/ 9B10A9H3) the produced antibodies were harvested in a period of two to three days. Mean production was 20 to 23 mg/week for 5A10E7F11H4 and 73 to 100 mg/week for 9B10A9H3 cell lines in this system (Figures 1A and 1B).

### (B) Quantitation of Mabs

Sandwich-type DELFIA was very sensitive the theoretical sensitivity being below 0.25 ng/ml for the Mabs of the present invention. Although the sensitivity was convenient for quantitation of Mabs produced in cell culture supernatants the assay was also practical for quantitation of Mabs produced in vitro. The linear range reached from 0.25 ng/ml to 250 ng/ml-500 ng/ml (Figures 2 and 3). Intraassay variation was found to be very low. The degree of labelling influenced slightly to the assay through increasing the background values being optimal at level of about 4 Eu³⁺/PSA.

### (C) Specificity of antiPSA Mabs

The specificity of antiPSA Mabs was tested through fractionation of PSA mixture that contains PSA-ACT complex and free PSA in Ultrogel 44 column using 50 mmol/L Tris buffer pH 7.2 with 0.15 mol/L NaCl. The total PSA containing PSA and PSA-ACT (99kDa) was measured with the specific PSA kit (DELFIA™, Wallac). Free form of PSA (33 kDa) was measured using antiPSA 5A10E7F11H4 as catching antibody on the solid phase (Lövgren et al. 1984) and Eu-labelled antiPSA Mab that recognized both the free form and complexed form of PSA as detector antibody (the DELFIA™-kit reagent) (Figure 4).

### (D) Nature of the epitopes, sensitivity to denaturing conditions.

AntiPSA 5A10E7F11H4 and 9B10A9H3 antibodies were used to probe proteins blotted to PVDF membranes after agarose gel electrophoresis (Figure 5a) or SDS-PAGE (Figures 5b, 5c). Purified PSA was identified by the Mabs of the present invention whereas the PSA coupled to ACT produced a barely visible color reaction (Mabs of the present invention).

On the Western blots from SDS-PAGE of reduced samples antiPSA 9B10A9H3 Mab produced a weak barely discernible reaction with both the purified PSA and the PSA complexed to ACT (Figure 5b). AntiPSA 5A10E7F11H4 Mab gave identical results (data not shown).

On the Western blots from SDS-PAGE gels under non-reduced conditions purified PSA was identified by both of the Mabs 5A10E7F11H4 and 9B10A9H3 whereas PSA complexed to ACT produced a very weak but visible color reaction (Figure 5c).

It will be appreciated that the methods and compositions of the instant invention can be incorporated in the form of a variety of embodiements, only a few of which are disclosed herein. It will be apparent to the artisan that other embodiments exist and do not depart from the spirit of the invention. Thus, the described embodiments are illustrative and should not be construed as restrictive.

## Claims

1. The hybridoma cell line 5A10E7F11H4 having the identifying characteristics of ECACC 93031201.

2. A monoclonal antibody which binds to free prostate-specific antigen but not to prostate-specific antigen complexed to α₁-antichymotrypsin wherein said antibody is produced by the hybridoma cell line 5A10E7F11H4 having the identifying characteristics of ECACC 93031201.

3. The hybridoma cell line 9B10A9H3 having the identifying characteristics of ECACC 93031202.

4. A monoclonal antibody which binds to free prostate-specific antigen but not to prostate-specific antigen complexed to α₁-antichymotrypsin wherein said antibody is produced by the hybridoma cell line 9810A9H3 having the identifying characteristics of ECACC 93031202.

5. In a method for differentiating between benign prostatic hyperplasia and prostate cancer which comprises determining the ratio of free PSA : total PSA or free PSA : PSA complexed with α₁-antichymotrypsin or PSA complexed with α₁-antichymotrypsin : total PSA in serum, wherein the improvement comprises determining the free PSA with the monoclonal antibody of claim 2.

6. In a method for differentiating between benign prostatic hyperplasia and prostate cancer which comprises determining the ratio of free PSA : total PSA or free PSA : PSA complexed with α₁-antichymotrypsin or PSA complexed with α₁-antichymotrypsin : total PSA in serum, wherein the improvement comprises determining the free PSA with the monoclonal antibody of claim 4.
